# EUROPEAN PATENT SPECIFICATION

(11) **EP 1 311 702 B1**
(45) Date of publication and mention of the grant of the patent: **30.11.2005**
(21) Application number: 01922843.6
(22) Date of filing: 28.03.2001
(51) Int. Cl.: C12Q 1/00, G01N 27/28, G01N 27/30, G01N 33/487, G01N 27/403

(54) **CONTINUOUS PROCESS FOR MANUFACTURE OF DISPOSABLE ELECTRO-CHEMICAL SENSOR**
KONTINUIERLICHES VERFAHREN ZUR HERSTELLUNG VON ELEKTROCHEMISCHEN WEGWERFFÜHLERN
PROCEDE DE FABRICATION EN CONTINU DE CAPTEUR ELECTROCHIMQUE JETABLE

(30) Priority: 28.03.2000 US 537599
(43) Date of publication of application: 21.05.2003
(73) Proprietor: Diabetes Diagnostics, Inc., Waltham, MA 02453 (US)
(72) Inventor: DAVIES, Oliver, William, Hardwicke, Inverness Circus Inverness 1V2 4QP (GB); MCALEER, Jerome, Francis, Grove Wantage OX12 0NR (GB); YEUDALL, Robert, Malcolm, Inverness 1V2 4LX (GB)
(74) Representative: Mercer, Christopher Paul
(86) International application number: PCT/US2001/010097
(87) International publication number: WO 2001/073109

(56) References cited:
- WO-A-98/43075
- US-A- 4 218 421
- US-A- 5 679 311

## Description

### BACKGROUND OF THE INVENTION

This application relates to electrochemical sensors useful for detection and/or quantification of a target analyte in a sample.

Disposable electrochemical sensors for monitoring of target analytes in blood or urine are well known. In particular, electrochemical measurement of the amount of glucose in a small amount of blood using disposable electrochemical sensors and small, portable meters has become a mainstay of many diabetics. These home-use systems permit routine measurements and provide the diabetic with an increased ability to self-manage his or her condition.

The disposable electrochemical sensors used in these devices are generally formed as a series of patterned layers supported on a substrate. Mass production of these devices has been carried out by screen printing and other deposition processes, with the multiple layers making up the device being deposited *seriatim* in a batch process.

WO98/430 discloses a method of manufacturing electrochemical sensors by rotogravure printing of electrically conductive ink to form one or more electrodes on a flexible web. A cylinder coated with a covering defining the shape of the electrodes to be printed may be used.

Manufacture of disposable electrochemical sensors by these techniques has several drawbacks. First, operation in batch mode is fundamentally inefficient. Multiple steps in the process requires the use of multiple print lines, one for each layer in the device. Not only does this increase the capital expense for the manufacturing equipment it also introduces multiple opportunities for process variation such as variable delays and storage conditions between print steps, as well as variations in the process itself such as registration drift between different process stations. Such process variations can result in poor calibration of some sensor batches resulting in potentially erroneous reading when the electrodes are used.

A potential second drawback arises from a characteristic inherent to screen printing, namely the thickness of the deposited layers. Standard screen printing processes can be used to deposit layers from 1 to 100 µm in thickness. Heat-cured resins can be used to obtain thinner layers of less than 1 µm in thickness. For printing electrodes, the capability of screen printing to produce layers with these dimensions is beneficial, since the thicker print has greater conductivity. For reagent layers, for example layers of enzymes which are utilized in many disposable electrochemical reactions, however, thick layers are detrimental to the reliable operation of the device. Specifically, because the amount of signal generated by a device of this type depends on the inter-reaction of these reagents and the target analyte within a very narrow region at the electrode surface, the use of reagent layers which extend beyond this region reduces the measured signal by depleting inwardly migrating analyte before it can reach the measurement zone.

In view of these drawbacks, there is a need for a new approach to the manufacture of disposable electrochemical sensors. It is an object of the present invention to meet this need.

It is a further object of this invention to provide a method for manufacturing disposable electrochemical sensors which operates as a continuous process and which provides for deposition of thin reagent layers.

It is a further object of the invention to provide cassettes which incorporate spooled sensors, including spooled sensors manufactured using the method of the invention.

It is a further object of the invention to provide sensors which having an integrated sealing layer which is combination with the substrate produce a sealed sample receiving chamber, thereby protecting the reagents until time of use, which sensors can be made using the method of the invention.

### SUMMARY OF THE INVENTION

These and other objects of the invention are met by a method in accordance with the invention for manufacturing electrochemical sensors. The sensors comprises a substrate, an electrode layer and at least a first reagent layer. The method comprises the steps of transporting a continuous web of the substrate past at least two cylinder screen printing stations, and printing the electrode layer and the first reagent layer on the substrate. One of the print stations prints the electrode layer on the continuous web of substrate, and the other of the print stations prints the first reagent layer on the continuous web of substrate as it is transported past the print stations. Additional print stations may be included for the printing of insulation layers, glue prints and the like. The order of printing will depend on the structure desired for the sensor, although the electrode layer(s) will frequently be deposited before the reagent layer(s).

### BRIEF DESCRIPTION OF THE FIGURES

Fig. 1A and 1B show two alternative deposition patterns useful in the method of the invention;
Figs. 2A and 2B show an exemplary electrochemical sensor which can be manufactured using the method of the invention;
Fig. 3 shows a schematic view of an apparatus for practicing the method of the invention;
Fig. 4 shows post-processing of a web printed with sensors to produce sensor spools;
Figs 5A and 5B shows cassettes useful with the sensor spool of Fig. 4, Fig. 5C shows a meter in combination with the cassette of Fig. 5A;
Fig. 6 shows an alternative embodiment of a sensor which can be manufactured using the method of the invention;
Figs. 7A and B shows a further alternative embodiment of a sensor which can be manufactured using the method of the invention; and
Figs. 8 A, B and C shows the application of a sealing layer to a ribbon of test strips in accordance with the invention.

### DETAILED DESCRIPTION OF THE INVENTION

The present invention provides a method for manufacturing electrochemical sensors using a continuous web of substrate transported past a plurality of printing stations for deposition of various layers making up the sensor. The method can be used for making sensors which are directed to any electrochemically-detectable analyte.

Exemplary analytes of particular commercial significance for which sensors can be made using the method include; glucose, fructosamine, HbAIC, lactate, cholesterol, alcohol and ketones.

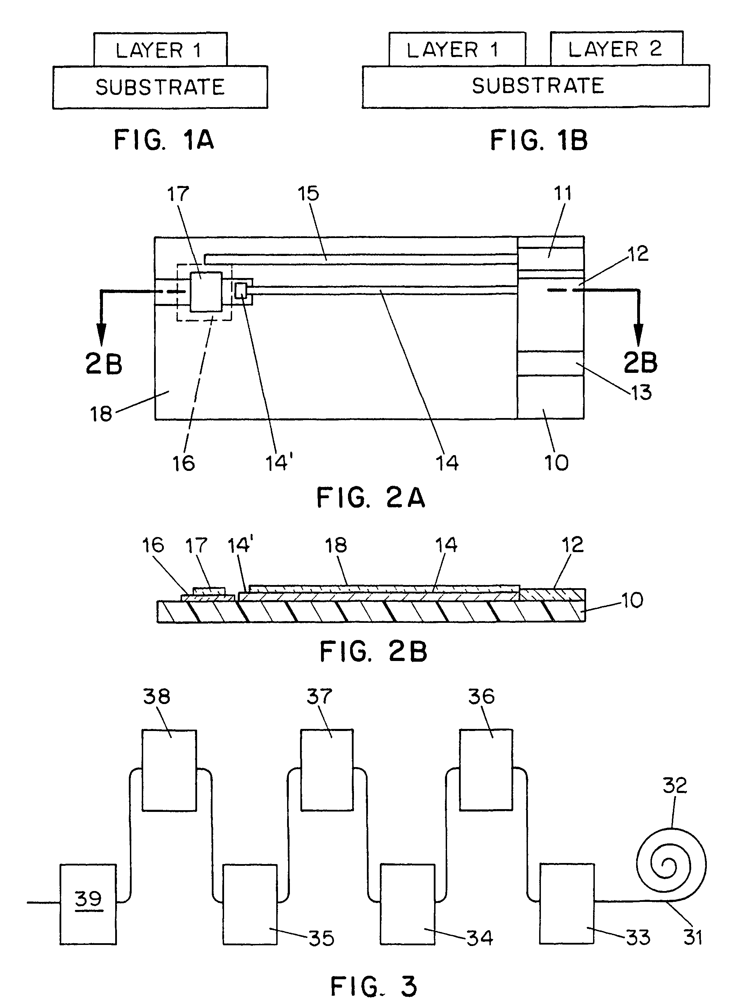

The specific structure of the electrochemical sensor will depend on the nature of the analyte. In general, however, each device will include an electrode layer and at least one reagent layer deposited on a substrate. As used in the specification and claims hereof, the term "layer" refers to a coating applied to all or part of the surface of the substrate. A layer is considered to be "applied to" or "printed on" the surface of the substrate when it is applied directly to the substrate or the surface of a layer or layers previously applied to the substrate. Thus, deposition of two layers on the substrate may result in a three layer sandwich (substrate, layer 1, and layer 2) as shown in Fig. 1A or in the deposition of two parallel tracks as shown in Fig. 1B, as well as intermediate configurations with partial overlap.

In the method of the invention, the electrochemical sensors are printed in a linear array, or as a plurality of parallel linear arrays onto a flexible web substrate. As discussed below, this web may be processed by cutting it into ribbons after the formation . As used in the specification and claims of this application, the term "ribbon" refers to a portion of the printed web which has been formed by cutting the web in either or both of the longitudinal and tranverse directions, and which has a plurality of electrochemical sensors printed thereon.

Figs. 2A and 2B show the structure of an electrochemical sensors for detection of glucose in accordance with in the invention. On the substrate 10 are placed a conductive base layer 16, a working electrode track 15, a reference electrode track 14, and conductive contacts 11, 12, and 13. An insulating mask 18 is then formed, leaving a portion of the conductive base layer 16, and the contacts 11, 12 and 13 exposed. A reagent layer of a working coating 17, for example a mixture of glucose oxidase and a redox mediator, is then applied over the insulating mask 18 to make contact with conductive base layer 16. Additional reagent layers can be applied over working coating 18 if desired. For example, the enzyme and the redox mediator can be applied in separate layers.

It will be appreciated that the specific structure shown in Figs. 2A and 2B is merely exemplary and that the method of the invention can be used to manufacture electrochemical sensors for a wide variety of analytes and using a wide variety of electrode/reagent configurations. Exemplary sensors which could be manufactured using the method of the invention include those disclosed in European Patent No. 0 127 958, and US Patents Nos. 5,141,868, 5,286,362, 5,288,636, and 5,437,999.

Fig. 3 shows a schematic view of an apparatus for practicing the invention. A running web of substrate 31 is provided on a feed roll 32 and is transported over a plurality of print stations 33, 34, and 35, each of which prints a different layer onto the substrate. The number of print stations can be any number and will depend on the number of layers required for the particular device being manufactured. Between successive print stations, the web is preferably transported through a dryer 36, 37, and 38 (for example a forced hot air or infra-red dryer), to dry each layer before proceeding to the deposition of the next. After, the final dryer 38, the printed web is collected on a take up roll or introduced directly into a post-processing apparatus 39.

While the most efficient embodiments of the invention will generally use a plurality of print stations as illustrated in Fig. 3 for the printing of different materials, it will be appreciated that many of the advantages of the invention can be achieved with a process in which a single print station is used several times with different print reagents. In particular, benefits of increased throughput and improved print registration are obtained when using the same print station multiple times. Thus, as used in the specification and claims of this application, the phrase "at least two print stations" refers both to embodiments in which two or more distinct print stations are employed and to embodiments in which a common print station is used in several passes to print the required materials onto the substrate.

As noted above, one of the most important parameters to control when printing the various layers of a bionsesor is the thickness of the deposited layer, particularly with respect to the reagent layer. The thickness of the printed layer is influenced by various factors, including the angle at which the substrate and the screen are separated. In a conventional card printing process, where the substrate is presented as individual cards on a flat table, this angle varies as the squeegee moves across the screen, leading to variations in thickness and therefore to variations in the sensor response across the card. To minimize this source of variation, the print stations used in the method of the present invention makes use of cylinder screen printing.

In cylinder screen printing, a flexible substrate is presented to the underside of a screen bearing the desired image using a cylindrical roller and moves synchronously with the squeegee. Unlike conventional printing, where the screen moves away from a stationary substrate, in this process the moving substrate is pulled away from the screen. This allows a constant separation angle to be maintained, so that a uniform thickness of deposit is achieved. What is more, the contact angle, and thus the print thickness can be optimized by choosing the appropriate point of contact. By appropriate optimization, the process can be engineered so that the ink is pulled out of the screen and transferred to the substrate much more efficiently. This sharper "peel off" leads to much improved print accuracy, allowing a finer detail print. Therefore smaller electrodes can be printed and smaller overall sensors can be achieved.

The post-processing apparatus 39 may perform any of a variety of treatments, or combinations of treatments on the printed web. For example, the post processing apparatus may apply a cover over the electrochemical devices by laminating a second continuous web to the printed substrate. The post-processing apparatus may also cut the printed web into smaller segments. To produce individual electrochemical devices of the type generally employed in known hand-held glucose meters, this cutting process would generally involve cutting the web in two directions, longitudinally and laterally. The use of continuous web technology offers the opportunity to make electrochemical sensors with different configurations which offer advantages for packaging and use.

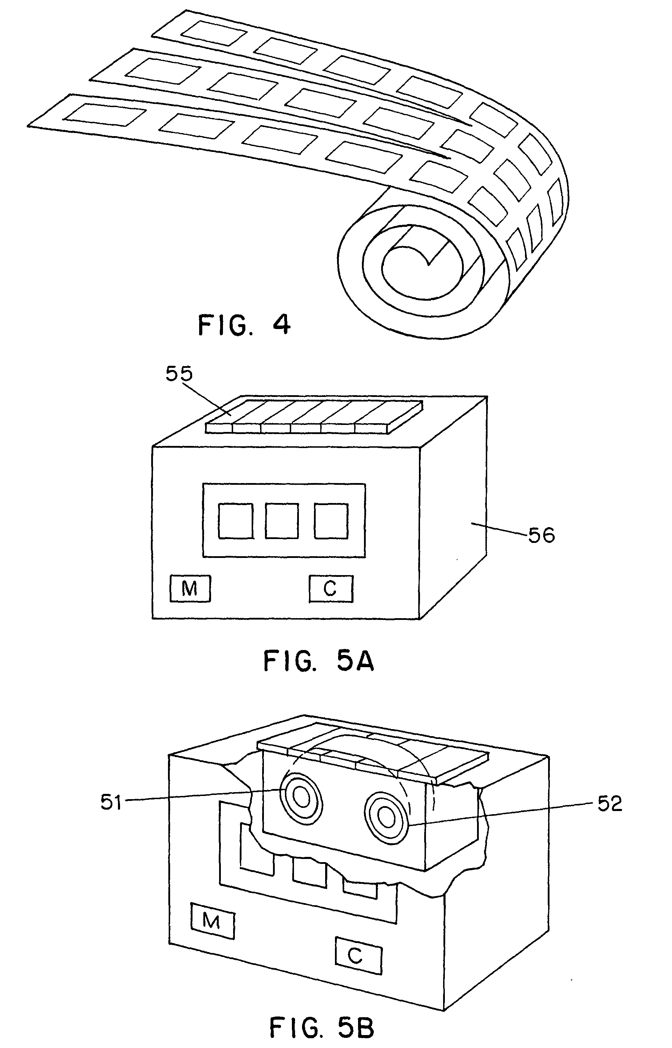

As shown in Fig. 4**,** the printed web can be cut into a plurality of longitudinal ribbons, each one sensor wide. These ribbons can in turn be cut into shorter ribbons of convenient lengths, for example, 10, 25, 50 or even 100 sensors. These ribbons may be rolled into spools and packaged into a cassette which is inserted into a meter (Fig. 5A). Alternatively, a short ribbon of say 5 strips can be prepared to provide enough sensors for one normal day of testing. For this length, a cassette is probably not necessary, although it could be provided if desired. In either case, the sensors are used one and a time, and moved into the appropriate position at the time of use. Preferably, this movement is accomplished by a meter-resident mechanism, which also prevents used strips from being drawn back inside the meter.

The use of spooled ribbons with multiple sensors has substantial advantages over the known systems using single electrochemical sensors. Because the spooled electrochemical devices are packaged inside a cassette, they are less susceptible to damage. Further, since the spool of devices is a continuous strip and is not intended to be removed from the cassette prior to use, there is less likelihood that a sensor will be used with the wrong calibration codes. The risk of erroneous calibration values can be further reduced if the cassette and the meter interact to provide calibration values for the sensors contained within the cassette. Interactions of this type are described for individual sensor devices in International Patent Publication No. WO97/29847.

A further advantage of continuous spools of electrochemical sensors is the ability to make each individual smaller. Much of the size of known individual sensors is driven by a requirement that the user be able to manipulate the sensor for insertion in the meter. Use of a continuous spool of sensors eliminates these constraints on the size of the device since the user will be manipulating the cassette or ribbon of electrochemical sensors which will be significantly easier to handle than individual strips. Thus, the present invention permits the fabrication of smaller and therefore more economical devices.

If it is desired to separate used devices from the spool, a cutter may be incorporated into the meter or into the cassette. A cutter of this type is disclosed in US Patent No. 5,525,297, although other configurations could be employed.

Fig. 5B shows variation of the meter of Fig. 5A. In this case, the cassette includes a take up mechanism such that the sensor spool is transferred from a feed spool 51 to a take up spool 52 as it is used. This makes the entire cassette system self-contained and eliminates the need to dispose of individual sensors which have frequently been contaminated with blood.

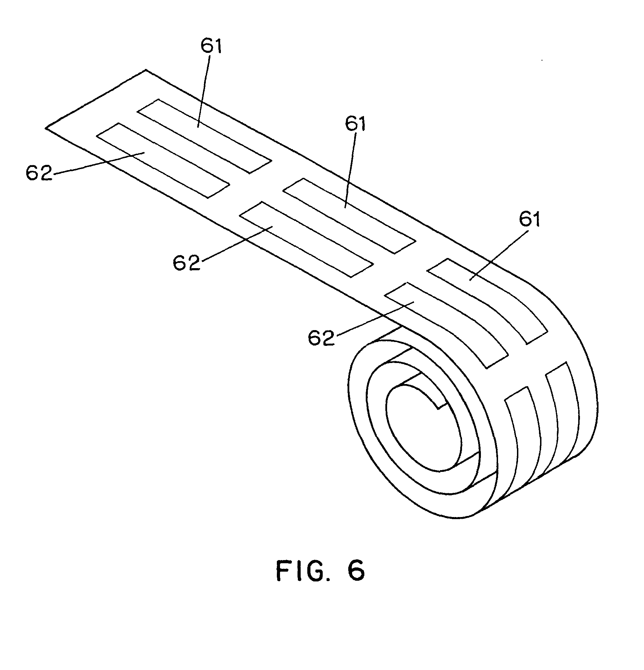

The method of the invention can also be used to produce sensor spools having parallel arrays of sensors of different types. Thus, as shown in Fig. 6, a sensor strip could be prepared in which sensors of a first type, 61 are disposed alongside sensors of a second type, 62. By providing separate contacts and analysis circuitry for each sensor, two values can be determined simultaneously in the same meter with the same sample. Suitable analyte pairs include glucose and glycosylated hemoglobin; and LDL and HDL. Two different sensors measuring levels of the same analyte might also be employed to provide and internal check, or to increase the dynamic range of the strip.

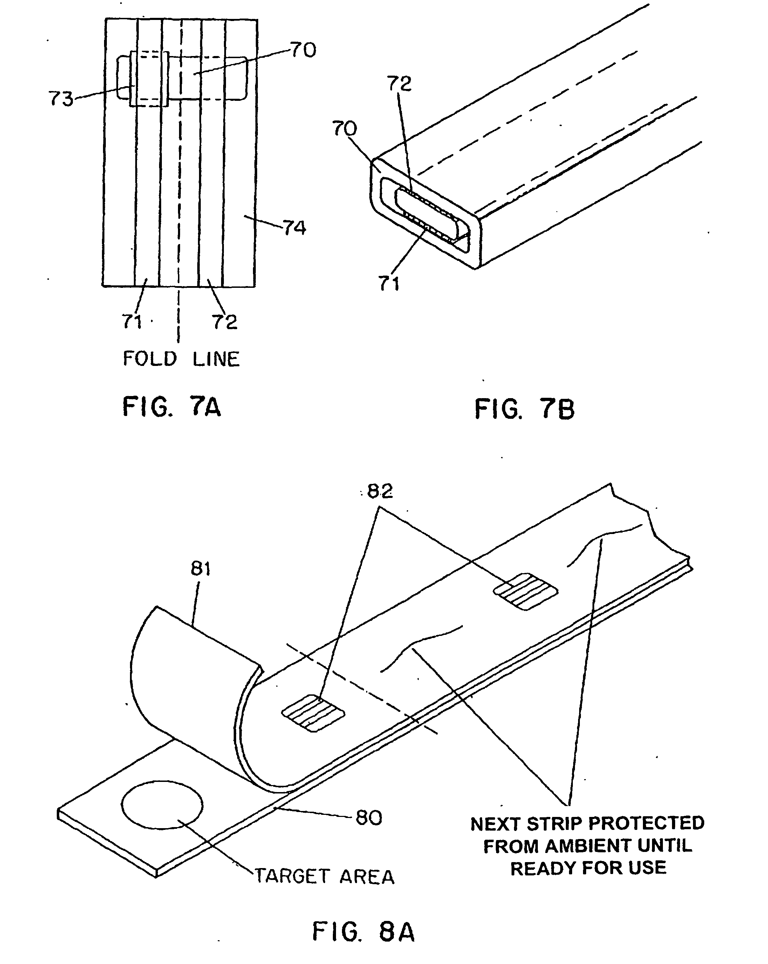

The method of the invention also facilitates the manufacture of sensors having structures which cannot be conveniently produced using conventional batch processing. For example, as shown in Figs. 7A and 7B, a device can be manufactured by depositing parallel conductive tracks 71 and 72; reagent layer(s) 73 and an insulation layer 74 on a substrate 70. The substrate is then folded along a fold line disposed between the two conductive tracks to produce a sensor in which two co-facial electrodes are separated by a reagent layer. An electrode geometry of this type is beneficial because the voltage drop due to solution resistance is low as a result of the thin layer of solution separating the electrodes. In contrast, in a conventional device with coplanar electrodes, the use of a thin layer of solution results in a substantial voltage drop along the length of the cell and concomitant uneven current distribution. Furthermore the device of Figs. 7A and 7B can be cut across the deposited reagent to produce a very low volume chamber for sample analysis which further improves the performance of the device.

As is apparent from the foregoing discussion, the method of the present invention provides a very versatile approach for manufacture of electrochemical sensors. The following discussion of suitable materials which can be used in the method of the invention is intended to further exemplify this versatility and not to limit the scope of the invention which is defined by the claims.

The substrate used in the method of the invention may be any dimensionally stable material of sufficient flexibility to permit its transport through an apparatus of the type shown generally in Fig. 3. In general the substrate will be an electrical insulator, although this is not necessary if a layer of insulation is deposited between the substrate and the electrodes. The substrate should also be chemically compatible with the materials which will be used in the printing of any given sensor. This means that the substrate should not significantly react with or be degraded by these materials, although a reasonably stable print image does need to be formed. Specific examples of suitable materials include polycarbonate and polyester.

The electrodes may be formed of any conductive material which can be deposited in patterns in a continuous printing process. This would include carbon electrodes and electrodes formed from platinized carbon, gold, silver, and mixtures of silver and silver chloride.

Insulation layers are deposited as appropriate to define the sample analysis volume and to avoid a short circuiting of the sensor. Insulating materials which can be printed are suitable, including for example polyester-based inks.

The selection of the constituents of the reagent layer(s) will depend on the target analyte. For detection of glucose, the reagent layer(s) will suitably include an enzyme capable of oxidizing glucose, and a mediator compound which transfers electrons from the enzyme to the electrode resulting in a measurable current when glucose is present. Representative mediator compounds include ferricyanide, metallocene compounds such as ferrocene, quinones, phenazinium salts, redox indicator DCPIP, and imidazole-substituted osmium compounds. The reagents appropriate to other types of sensors will be apparent to persons skilled in the art.

One of the limitations of any device in which multiple test elements are stored within a test device is that the elements must be made stable for the expected lifetime of the test elements within the test device. In general, for electrochemical sensor strips, this means providing a moisture-proof and air-tight environment for unused sensor strips. This can be accomplished through the design of the cassette and associated meter, or it may be accomplished by adding a sealing layer to the test ribbon so that individual test strips are individually sealed and protected from moisture.

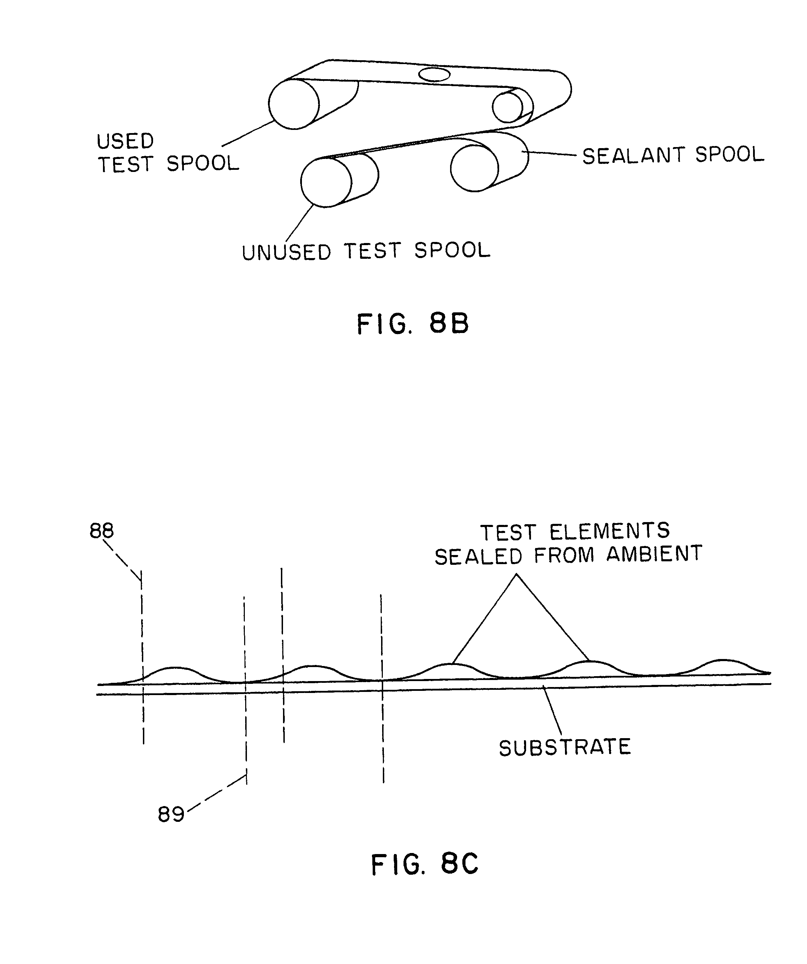

Figs. 8 A-C relate to ribbons of test strips with a sealing layer. Fig. 8A shows a composite structure comprising a lower layer ribbon of test strips 80 and an upper sealing layer 81. The upper sealing layer 81 is shown partially peeled back to expose the first test element. The upper layer contains apertures 82 through which electrical contact with the underlying test strip can be made. The sealing layer 81 is typically attached to the ribbon 80 using a hot melt or pressure-sensitive adhesive. The meter employed with the sealed test strip ribbon of Fig. 8A would include a mechanism, such as a knife blade, for peeling back the sealing layer 81 to expose the target area of a strip that is about to be used. After use, the used test strip and the peeled back sealing layer may be cut away from the unused portion of the ribbon, for example using a cutter blade integral to the cassette. The used strips and peeled off sealing layer might also be rolled up onto take-up spools within a cassette as shown in Fig. 8B, thus avoiding the need for a user to contact used strips directly.

Fig. 8C shows a variation on the structure of Fig. 8C. In this case, the sealant layer serves as one wall of the test strip sample chamber. This geometry has certain advantages, notably that evaporative cooling of the sample (which can lead to erroneously low readings) is reduced. To prepare a test strip on a ribbon of this type for use, a cut is made which opens the end ofa chamber formed by the sealing layer 81 and the test strip ribbon 80. In Fig. 8C, separate cut line-types 88 and 89 are shown for separating used devices and for opening a new device, respectively. These cuts can be made at the same type or at different times.

## Claims

1. A method for manufacturing electrochemical sensors comprising a substrate, an electrode layer and at least a first reagent layer, said method comprising the steps of transporting a continuous web of the substrate (31) past at least two print stations (33, 34, 35) and printing the electrode layer (16) and the first reagent layer (17) on the substrate, one of said print stations (33) printing the electrode layer (16) on the continuous web of substrate (31) and the other said print stations (34) printing the first reagent layer (17) on the continuous web of substrate (31) as it is transported past the print stations, **characterised in that** the print stations (33, 34, 35) are cylinder screen printing stations.

2. The method according to claim 1, wherein the electrochemical sensors detect glucose.

3. The method according to claim 2, wherein the first reagent layer (17) comprises glucose oxidase.

4. The method according to any of the preceding claims, wherein the disposable electrochemical sensors further comprise a second reagent layer which is deposited on the continuous web substrate by a third print station.

5. The method according to claim 4, wherein the second reagent layer comprises an electron transfer mediator.

6. The method according to claim 5, wherein the electron transfer mediator is ferricyanide.

7. The method according to any of the preceding claims, wherein the print stations (33, 34) which print the electrode layer (16) and the first reagent layer (17) are separate and distinct print stations.

8. The method according to claim 7, wherein the continuous web of substrate (31) is transported between the print stations (33, 34, 35) in a continuous process.

9. The method according to claim 7 or 8, wherein the continuous web of substrate (31) is transported through a dryer (36, 37, 38) between the print stations (33, 34, 35) which print the electrode layer (16) and the first reagent layer (17).

10. The method according to claim 9, wherein the dryer (36, 37, 38) is an infra-red dryer.

11. The method according to any of the preceding claims, further comprising a sealing post-processing step applied to the web after printing of the electrochemical sensors in which a sealing layer (81) is applied over the electrochemical sensors.

12. The method according to claim 11, wherein the sealing layer (81) and the web (31) having the electrochemical sensors printed thereon cooperate to form a sample-receiving chamber which can be opened by cutting the end of a sensor.

13. The method according to any of the preceding claims, further comprising a cutting post-processing step applied to the web after printing of the electrochemical sensors in which the web is cut into ribbons, each ribbon containing a plurality of sensors.

14. The method according to claim 13, wherein each ribbon contains from 5 to 100 sensors.

15. A cassette comprising a case and a ribbon disposed within the case on which a plurality of disposable electrochemical sensors are provided, wherein the sensors are manufactured in accordance with the method of any of claims 1-14.

16. The cassette according to claim 15, wherein the electrochemical sensors are for the detection of glucose.

17. An electrochemical sensor produced by the method according to any of claims 1-14.

18. The electrochemical sensor according to claim 17 for the detection of an analyte such as glucose, wherein the method comprises printing a sensor on a substrate and covering the sensor by a sealing layer, said substrate and sealing layer cooperating to form a sealed sample-receiving chamber, and wherein the method further comprises cutting the sealed sample-receiving chamber in use to produce an opening to the sample-receiving for the introduction of analyte to the sample.

## Patentansprüche

1. Verfahren zum Herstellen elektrochemischer Sensoren, die ein Substrat, eine Elektrodenschicht und wenigstens eine erste Reagenzschicht aufweisen, wobei das Verfahren die Schritte Transportieren einer kontinuierlichen Lage des Substrats (31) hinter wenigstens zwei Druckstationen (33, 34, 35) und Aufdrucken der Elektrodenschicht (16) und der ersten Reagenzschicht (17) auf das Substrat, wobei eine der Druckstationen die Elektrodenschicht (16) auf die kontinuierliche Lage des Substrats (31) druckt und die andere der Druckstationen (34) die erste Reagenzschicht (17) auf die kontinuierliche Lage des Substrats (31) druckt, wenn sie hinter die Druckstationen transportiert wird, umfaßt, **dadurch gekennzeichnet, daß** die Druckstationen (33, 34, 35) Zylinder-Siebdruckstationen sind.

2. Verfahren nach Anspruch 1, bei dem die elektrochemischen Sensoren Glukose erfassen.

3. Verfahren nach Anspruch 2, bei dem die erste Reagenzschicht (17) Glukoseoxidase aufweist.

4. Verfahren nach einem der vorangehenden Ansprüche, bei dem die entsorgbaren elektrochemischen Sensoren weiterhin eine zweite Reagenzschicht aufweisen, die auf der kontinuierlichen Substratlage durch eine dritte Druckstation abgelegt wird.

5. Verfahren nach Anspruch 4, bei dem die zweite Reagenzschicht einen Vermittler für den Elektronenübergang aufweist.

6. Verfahren nach Anspruch 5, bei dem der Vermittler für den Elektronenübergang Hexacyanoferrat (III) ist.

7. Verfahren nach einem der vorangehenden Ansprüche, bei dem die Druckstationen (33, 34), welche die Elektrodenschicht (16) und die erste Reagenzschicht (17) drucken, getrennte und unterschiedliche Druckstationen sind.

8. Verfahren nach Anspruch 7, bei dem die kontinuierliche Lage des Substrats (31) zwischen den Druckstationen (33, 34, 35) in einem kontinuierlichen Prozeß transportiert wird.

9. Verfahren nach Anspruch 7 oder 8, bei dem die kontinuierliche Lage des Substrats (31) durch einen Trockner (36, 37, 38) zwischen den Druckstationen (33, 34, 35), welche die Elektrodenschicht (16) und die erste Reagenzschicht (17) drucken, transportiert wird.

10. Verfahren nach Anspruch 9, bei dem der Trockner (36, 37, 38) ein Infrarot-Trockner ist.

11. Verfahren nach einem der vorangehenden Ansprüche, das weiterhin einen Schritt des Versiegelns nach der Bearbeitung aufweist, der auf die Lage angewendet wird, nachdem die elektrochemischen Sensoren gedruckt sind, bei dem eine Versiegelungsschicht (81) über die elektrochemischen Sensoren gelegt wird.

12. Verfahren nach Anspruch 11, bei dem die Versiegelungsschicht (81) und die Lage (31) mit den darauf gedruckten elektrochemischen Sensoren zusammenwirken, um eine probenaufnehmende Kammer zu bilden, die geöffnet werden kann, indem das Ende eines Sensors geschnitten wird.

13. Verfahren nach einem der vorangehenden Ansprüche, das weiterhin einen Schritt des Schneidens nach der Bearbeitung aufweist, der bei der Lage nach dem Drucken der elektrochemischen Sensoren angewendet wird, bei dem die Lage in Bänder geschnitten wird, wobei jedes Band eine Vielzahl von Sensoren enthält.

14. Verfahren nach Anspruch 13, bei dem jedes Band 5 bis 100 Sensoren enthält.

15. Kassette, mit einem Gehäuse und einem Band, das innerhalb des Gehäuses angeordnet ist, auf dem eine Vielzahl entsorgbarer elektrochemischer Sensoren vorgesehen ist, wobei die Sensoren entsprechend dem Verfahren nach einem der Ansprüche 1 bis 14 hergestellt sind.

16. Kassette nach Anspruch 15, bei der die elektrochemischen Sensoren für das Erfassen von Glukose vorgesehen sind.

17. Elektrochemischer Sensor, hergestellt durch das Verfahren nach einem der Ansprüche 1 bis 14.

18. Elektrochemischer Sensor nach Anspruch 17 für die Erfassung einer analytischen Substanz, so wie Glukose, wobei das Verfahren das Drucken eines Sensors auf ein Substrat und das Abdecken des Sensors durch eine Versiegelungsschicht aufweist, wobei das Substrat und die Versiegelungsschicht zusammenwirken, um eine versiegelte probenaufnehmende Kammer zu bilden, und wobei das Verfahren weiterhin das Schneiden der versiegelten probenaufnehmenden Kammer bei der Verwendung aufweist, um eine Öffnung in dem Probenaufnehmer für das Einführen der analytischen Substanz zur Probe zu erzeugen.

## Revendications

1. Procédé de fabrication de capteurs électrochimiques comprenant un substrat, une couche de type électrode et au moins une première couche de réactif, lequel procédé comprend les étapes consistant à transporter un voile continu de substrat (31) au-delà d'au moins deux stations d'impression (33, 34, 35) et imprimer la couche de type électrode (16) ainsi que la première couche de réactif (17) sur le substrat, l'une desdites stations d'impression (33) imprimant la couche de type électrode (16) sur le voile continu de substrat (31) tandis que lesdites autres stations d'impression (34) impriment la première couche de réactif (17) sur le voile continu de substrat (31) à mesure que celui-ci est transporté au-delà des stations d'impression, **caractérisé en ce que** les stations d'impressions (33, 34, 35) consistent en des stations de sérigraphie à cylindres.

2. Procédé selon la revendication 1, dans lequel les capteurs électrochimiques détectent le glucose.

3. Procédé selon la revendication 2, dans lequel la première couche de réactif (17) comprend de l'oxydase de glucose.

4. Procédé selon l'une quelconque des revendications précédentes, dans lequel les capteurs électrochimiques jetables comprennent en outre une seconde couche de réactif qui est déposée sur le substrat de type voile continu par une troisième station d'impression.

5. Procédé selon la revendication 4, dans lequel la seconde couche de réactif comprend un médiateur de transfert d'électrons.

6. Procédé selon la revendication 5, dans lequel le médiateur de transfert d'électrons consiste en du ferricyanure.

7. Procédé selon l'une quelconque des revendications précédentes, dans lequel les stations d'impression (33, 34) imprimant la couche de type électrode (16) et la première couche de réactif (17) consistent en des stations d'impression distinctes et séparées.

8. Procédé selon la revendication 7, dans lequel le voile continu de substrat (31) est transporté entre les stations d'impression (33, 34, 35) selon un processus continu.

9. Procédé selon la revendication 7 ou 8, dans lequel le voile continu de substrat (31) est transporté à travers un séchoir (36, 37, 38) situé entre les stations d'impression (33, 34, 35) qui impriment la couche de type électrode (16) et la première couche de réactif (17).

10. Procédé selon la revendication 9, dans lequel le séchoir (36, 37, 38) consiste en un séchoir à infrarouge.

11. Procédé selon l'une quelconque des revendications précédentes, comprenant en outre une étape de traitement ultérieur de scellage que l'on applique au voile après l'impression des capteurs électrochimiques, et dans laquelle une couche de scellage (81) est appliquée sur les capteurs électrochimiques.

12. Procédé selon la revendication 11, dans lequel la couche de scellage (81) et le voile (31) sur lequel sont imprimés les capteurs électrochimiques entrent en interaction de manière à former une chambre de réception d'échantillon qui peut être ouverte en découpant l'extrémité d'un capteur.

13. Procédé selon l'une quelconque des revendications précédentes, comprenant en outre une étape de traitement ultérieur de découpage que l'on applique au voile après l'impression des capteurs électrochimiques, et dans laquelle le voile est découpé en rubans, chaque ruban comprenant plusieurs capteurs.

14. Procédé selon la revendication 13, dans lequel chaque ruban comprend de 5 à 100 capteurs.

15. Cassette comprenant un boîtier ainsi qu'un ruban qui est disposé dans le boîtier et sur lequel se trouvent plusieurs capteurs électrochimiques jetables, lesquels capteurs sont fabriqués selon le procédé de l'une quelconque des revendications 1 à 14.

16. Cassette selon la revendication 15, dans laquelle les capteurs électrochimiques sont destinés à détecter le glucose.

17. Capteur électrochimique fabriqué selon le procédé de l'une quelconque des revendications 1 à 14.

18. Capteur électrochimique selon la revendication 17, servant à détecter un analyte tel que du glucose, le procédé consistant à imprimer un capteur sur un substrat et à couvrir le capteur par une couche de scellage, ledit substrat et ladite couche de scellage entrant en interaction de manière à former une chambre de réception d'échantillon scellée, et le procédé consistant en outre à découper la chambre de réception d'échantillon scellée lors de l'utilisation de manière à produire une ouverture de réception d'échantillon afin d'introduire un analyte vers l'échantillon.
